# EUROPEAN PATENT SPECIFICATION

(11) **EP 3 258 888 B1**
(45) Date of publication and mention of the grant of the patent: **02.04.2025**
(21) Application number: 16751978.4
(22) Date of filing: 16.02.2016
(51) Int. Cl.: A61F 2/38, A61F 2/36

(54) **CONTAINMENT BODY AND METHOD FOR MAKING A SPACER DEVICE COMPRISING SUCH CONTAINMENT BODY**
EINDÄMMUNGSKÖRPER UND VERFAHREN ZUR HERSTELLUNG EINER ABSTANDHALTERVORRICHTUNG MIT SOLCH EINEM EINDÄMMUNGSKÖRPER
CORPS DE CONFINEMENT ET PROCÉDÉ DE FABRICATION D'UN DISPOSITIF D'ÉCARTEMENT COMPRENANT UN TEL CORPS DE CONFINEMENT

(30) Priority: 16.02.2015 IT BO20150065
(43) Date of publication of application: 27.12.2017
(73) Proprietor: TECRES S.P.A., 37066 Sommacampagna (VR) (IT)
(72) Inventor: Magagnoli, Augusto, 48015 Cervia (Ravenna) (IT)
(74) Representative: Feltrinelli, Secondo Andrea
(86) International application number: PCT/IB2016/000135
(87) International publication number: WO 2016/132201

(56) References cited:
- US-A- 5 769 897
- US-A1- 2005 012 610
- US-A1- 2007 129 809
- US-A1- 2010 042 214
- US-A1- 2012 165 871

## Description

### TECHNICAL FIELD OF THE INVENTION

The present invention relates to a containment body for making a temporary and/or disposable spacer device for treating an infected bone seat or joint seat.

In particular, the aforesaid containment body constitutes, following the obtainment of the temporary and/or disposable spacer device, an integral part of the spacer device itself.

The present invention also relates to a temporary and/or disposable spacer device comprising such containment body and a method for making one such spacer device.

### STATE OF THE PRIOR ART

It is known that the prostheses implanted inside the human body can be subjected to infections.

In such event, the infected prosthesis must be removed from the implant site and, before the implant of a new prosthesis, the infection must be eradicated.

During such step, spacer devices are normally used for the purpose of substantially maintaining unchanged the shape of the bone seat or of the joint seat in which the new prosthesis will be implanted.

Such procedure is known as "two-step treatment" for the removal of an infected prosthesis and the implant of a new prosthesis.

Such spacer devices nevertheless have limits regarding the quantity or type of pharmaceutical or medical substance that can be released or regarding the possibility to ensure a substantially uniform release of such substance. Once again, it is observed that, if a spacer device only has the outer surface porous, the quantity of pharmaceutical or medical substance that can be impregnated in the spacer device is limited by the depth and by the extension of the porous surface itself. In such case, the spacer device might not be able to ensure a release of the pharmaceutical or medical substance for a period of time equal to that necessary for the complete healing of the infected site.

Finally, there are spacer devices which are made of a biocompatible material which has pores over the entire volume occupied by the device itself.

Such spacers are, however, usually preloaded with a specific antibiotic or with a specific medical or pharmaceutical substance and therefore the surgeon is not free to select the type of drug to be used, hence is unable to adapt the drug itself to the actual needs of the patient.

There is therefore the need to provide a spacer device that ensures a uniform and constant release of the pharmaceutical or medical substance present through the entire outer surface of the spacer device itself, also for extended time periods.

There are then preformed spacer devices which are produced by casting antibiotic bone cement in a mold up to the hardening thereof, removing the mold and extracting the hardened spacer device, which is then subsequently worked or finished in accordance with the requirements.

Alternatively, the surgeon can himself make a spacer during the operating phase by using molds - usually made of silicone of suitable geometry - which are filled with antibiotic bone cement, to which a further antibiotic different from the first is possibly added. Once the polymerization has taken place, the surgeon extracts the spacer from the silicone mold, aided by the flexible nature of the silicone material, and then proceeds with the implant, also in this case possibly finishing the spacer if necessary.

There is therefore the need, for the surgeon, to be able to select the pharmaceutical or medical substance to be applied to the spacer device itself, so as to meet the specific needs of the patient.

Simultaneously, this possibility is associated with the need to provide, in any case, a temporary and/or disposable spacer device with predefined and correct shape and size, without the risk that the surgeon - having to make the spacer device directly *in situ* - will obtain a shape that is irregular or incompatible with the actual anatomic needs of the patient, or in any case to be finished and worked before implant.

Document US 2010/042214 A1 discloses an orthopaedic implant suitable to be implanted at a selected location of a body and configured for delivering at least one therapeutic agent to the body itself. The implant comprises a reservoir, configured for receiving the at least one therapeutic agent, and a plurality of channels, configured for conveying the at least one therapeutic agent from the reservoir to a treatment site. The implant can be a porous device. This document, however, fails to disclose a cavity delimited by a plurality of side walls, wherein at least one opening for accessing to the cavity is delimited by the side walls and wherein the base portion and the sidewalls have a thickness between 0.5 mm and 20 mm.

### OBJECTS OF THE INVENTION

The task of the present invention is to improve the state of the prior art.

In the scope of such technical task, one object of the present invention is to provide a containment body for forming a temporary and/or disposable spacer device for the substantially uniform release of at least one pharmaceutical or medical substance in a bone seat or joint seat to be treated.

Another object of the present invention is to provide a containment body for forming a temporary and/or disposable spacer device whose shape substantially corresponds to that of the infected bone seat or joint seat to be treated or to part thereof.

A further object of the present invention is to provide a containment body, for forming a temporary and/or disposable spacer device, which has a high mechanical resistance to the stresses to which it is subjected during use. In particular, if the containment body is provided for making a spacer device subjected to rubbing, as in the case of an joint of the human body, the same must be made of a material with a high resistance to abrasion.

In accordance with one aspect of the present invention, a containment body is provided for making a spacer device, according to claim 1.

In accordance with another aspect of the present invention, a spacer device is provided for treating a bone seat or joint seat comprising one such containment body, according to claim 8.

One such spacer device is provided for releasing a pharmaceutical or medical substance in a substantially uniform manner through the entire outer surface of the spacer device itself.

In addition, one such spacer device can be made in a manner such that the release of one such pharmaceutical or medical substance is ensured, even for long time periods.

In accordance with a further aspect of the present invention, a method is provided for making a spacer device, for treating a bone seat or joint seat, comprising a containment body, according to claim 14.

The dependent claims refer to preferred and advantageous embodiments of the invention.

### BRIEF DESCRIPTION OF THE DRAWINGS

Further characteristics and advantages of the present invention will be clearer from the detailed description of a preferred but not exclusive embodiment of a containment body for forming a spacer device, illustrated as a non-limiting example in the enclosed drawing tables, in which:
figure 1 is a schematic top perspective view of a containment body according to the present invention;
figure 2 is a schematic top perspective view of a further version of the containment body according to the present invention;
figure 3 is a schematic top perspective view of the containment body pursuant to figure 2 associated with a further component;
figure 4 is an exploded view of a temporary and disposable spacer device comprising a containment body according to the present invention coupled to a further component;
figure 5 is a perspective view of a further configuration of a temporary and/or disposable spacer device comprising a containment body according to the present invention;
figure 6 is a section view, along a plane of line VI-VI, of the temporary and/or disposable spacer device pursuant to figure 5;
figure 7 is a side view of a temporary and/or disposable spacer device according to the present invention sectioned in two halves, the latter being arranged side-by-side each other;
figure 8 is a top view of a half of the temporary and/or disposable spacer device pursuant to figure 7 positioned on a Petri dish.

### EMBODIMENTS OF THE INVENTION

With reference to the enclosed figures, a containment body for forming a spacer device, e.g. a temporary and/or disposable spacer device, is indicated overall with reference number 1.

More precisely, the containment body 1 is provided for making or forming a spacer device to be implanted in a bone seat or joint seat of the human body, typically in substitution of an infected prosthesis.

Such spacer device is defined "temporary" in the sense that, once its curative function has been completed, together with its maintenance of the space of the bone seat or joint seat, it will be removed from the zone in question and substituted for example with a permanent prosthesis.

For such purpose, the spacer device performs the function of maintenance of the joint spaces as well as of treatment of the bone infection by freeing a quantity of antibiotic in the infected zone. With regard to the latter aspect, the spacer is able to cure the infection underway by releasing antibiotic in a targeted manner and in infinitesimal quantities, while the application of even high doses of antibiotic, but with methods that do not provide for the use of spacers, e.g. washing the infected place with high-dosage antibiotics, does not allow obtaining the same results.

Studies conducted in the field have in fact shown that the bone tissue absorbs in a concentrated manner all the antibiotic molecules (even if only a few) daily released by the spacer. Naturally this is verified if the antibiotic is released by the spacer in contact with or adjacent to the bone tissue, in which case the quantity of antibiotic locally reaches the effective concentration for eradicating the infection. For this reason, it is essential that the spacer is extended over the entire area of the infection, thus intending that if the infected prosthesis is a long prosthesis, a long spacer will be used, and if the infected prosthesis is of short type, a short spacer will be used. If a short spacer is placed where a long prosthesis had previously been implanted, part of the bone would not be treated with antibiotic, thus allowing the free bacteria to proliferate.

The containment body 1 has a shape such to be couplable, in a substantially complementary manner, to the bone seat or joint seat with which it must be constrained.

The containment body 1 appears as a substantially hollow casing provided with at least one opening through which a solidifiable filling material is introduced, in order to make a spacer device. In substance, the containment body 1 corresponds with the hollow figure - acting as a kind of mold - of the spacer device to be made (once the body itself is filled with a viscous fluid of the type which becomes solid or is solidifiable) and, in particular, forms the outer portion thereof.

One such temporary and/or disposable spacer device, once formed, thus comprises an outer portion, corresponding with the containment body 1, and an inner portion, comprising the filling material and, possibly, a reinforcing core or structure, as is better described hereinbelow.

The aforesaid temporary and disposable spacer device also constitutes the object of the present invention.

In the enclosed figures, by way of a non-limiting example, several possible configurations are illustrated of a containment body according to the present invention.

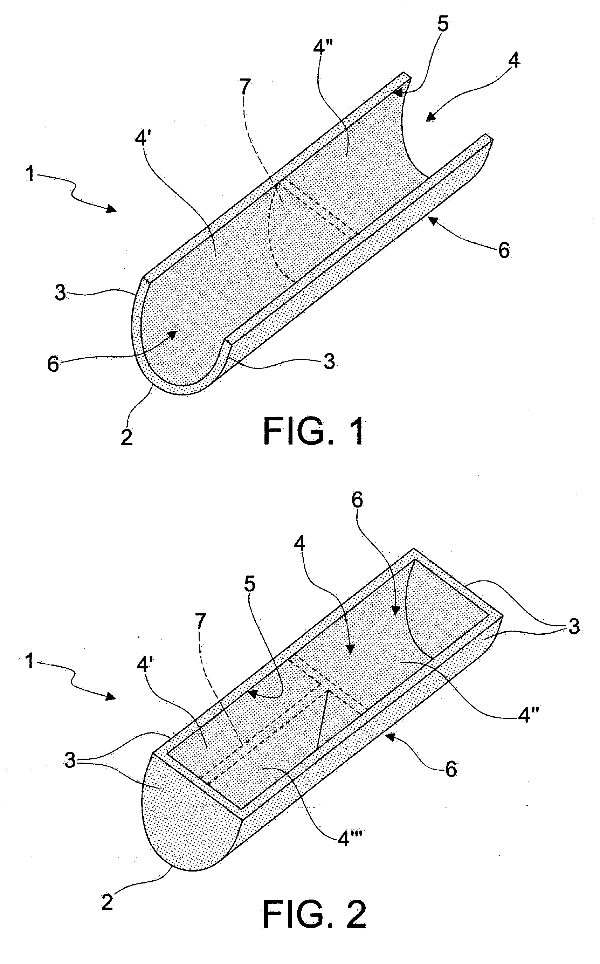

More in detail, by way of example, in the enclosed figures 1-3, several possible basic shapes of the containment body according to the present invention are schematized, in which the containment body itself has a substantially elongated shape.

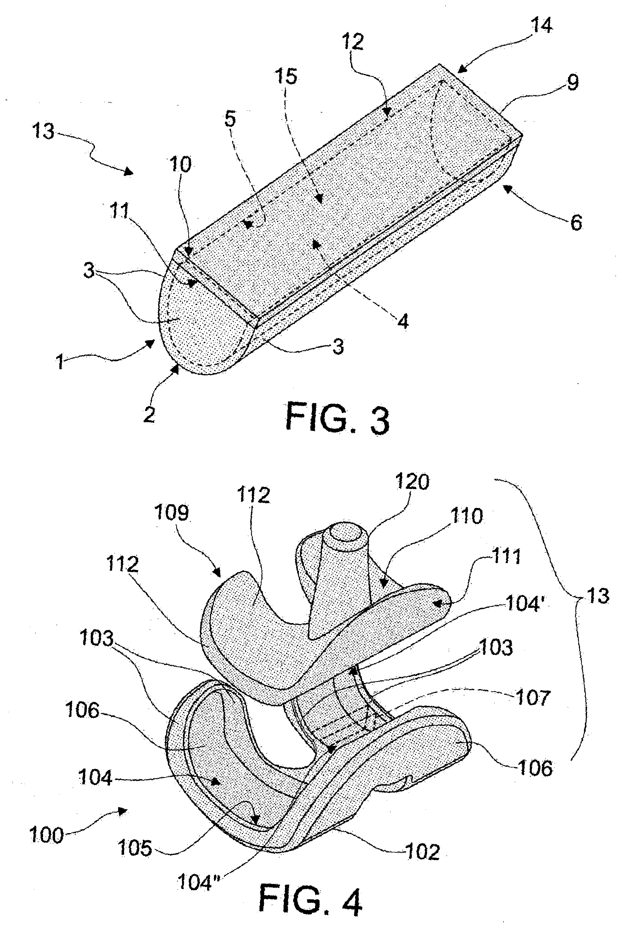

A further version of a containment body according to the present invention, provided for making a temporary and disposable spacer device constrainable to the femoral end of the knee joint, is illustrated in figure 4.

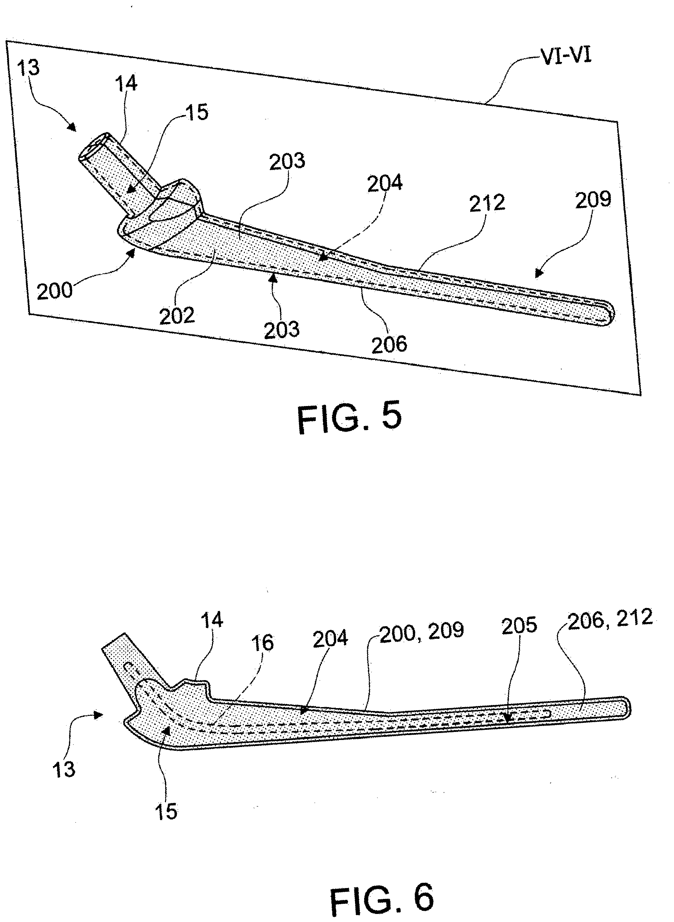

In figures 5 and 6, however, a further version of a containment body according to the present invention is depicted, provided for making a temporary and disposable spacer device for the articulation of the shoulder or hip.

Further configurations of the containment body according to the present invention are nevertheless possible, for making spacer devices shaped differently from that illustrated in the enclosed figures, without any limitation.

The containment body 1 for making a temporary and/or disposable spacer device comprises a base portion 2 and side walls 3 that extend from the base portion 2.

In particular, the base portion 2 and the side walls 3 together delimit a cavity 4.

At least one filling material of solidifiable type is housable in such cavity 4.

The base portion 2, along with the side walls 3, can be made of various thicknesses. Such thicknesses can be selected as a function of specific use requirements, such as the shape of the spacer device that one intends to make or the structural strength that the same must ensure.

According to the invention, the thickness of the base portion 2 and of the side walls is comprised between 0.5 mm a 20 mm, preferably between 0.5 and 4 mm.

As stated above, the containment body 1 has an opening 5 for access to the cavity 4. Such opening 5 is delimited by the side walls 3, in particular by their outer peripheral edges.

The opening 5, in one version of the invention, is opposite the base portion 2.

Through the opening 5, it is possible to introduce at least one filling material inside the containment body 1.

According to one version of the present invention, the side walls 3 perimetrically extend from the base portion (see figures 2 and 3).

In accordance with such version, the side walls 3 are continuous with each other, laterally delimiting the cavity 4 and, consequently, the opening 5.

The containment body 1 according to the present invention, and more precisely its base portion 2 and the side walls 3, is made of a biologically compatible material. According to one version of the present invention, the containment body 1 is made of porous material, as is better described hereinbelow.

The biologically compatible material constituting the containment body 1 can be selected from among plastic and/or polymer materials, such as polymethylmethacrylate (PMMA), polyethylene (PE), polyvinylchloride (PVC), polystyrene (PS), polyetheretherketone (PEEK), ultra-high molecular weight polyethylene (UHMWPE), high or low density polyethylene, or non-polymer materials, ceramics, metals, metal alloys, organometallic compounds and/or a combination thereof.

With reference to the biocompatible plastic materials, those preferred are sufficiently rigid at a temperature of about 36°C, i.e. physiological temperature, simultaneously ensuring the necessary mechanical performances according to the present invention and described in the present discussion.

Specifically, the aforesaid plastic materials can be selected from among thermoplastic polymers, such as acrylic resins, polyethylene, polypropylene, polyester, etcetera, thermoformable polymers, and other similar materials.

In one version of the present invention, the biologically compatible material is a bone cement with polymethylmethacrylate (PMMA) base. PMMA has the advantage of being perfectly welded with the filling material which, at least in one version of the invention, is bone cement, also comprising PMMA undergoing polymerization.

In one version of the invention, the aforesaid biologically compatible material initially lacks pharmaceutical or medical substances.

In a second version, the aforesaid biologically compatible material initially comprises at least one pharmaceutical or medical substance.

With regard instead to the filling material, typically the same can be bone cement.

The filling material can, in a further version of the invention, comprise an inorganic hydraulic cement or a biocompatible solid filling material.

According to a further version of the present invention, the filling material can be a ceramic cement, such as calcium sulfate known as plaster or CaSO₄, which in addition to solidifying in limited time periods, is also capable of releasing calcium ions.

According to another version, the filling material could have complex ceramic cement base, such ceramic cements based on calcium silicates and aluminates.

According to a further example that does not form part of the present invention, the filling material can be a liquid or a fluid whose viscosity is selected as a function of the duration of the period necessary for the treatment of the infected site, as is better described hereinbelow.

For example, the liquid or fluid could be able to elute through the containment body (or also through the closing body) for a time period variable between 1 and 12 months.

Further filling materials of biocompatible type are nevertheless usable, with respect to that described above, without departing from the protective scope of the present invention.

In one version of the invention, the filling material, which can be prepared by the surgeon during the operating procedure, lacks pharmaceutical or medical substances and the same can be added thereto based on the selection of the surgeon and on the needs of the patient.

In a further version of the invention, the aforesaid filling material can comprise at least one pharmaceutical or medical substance already arranged in the material that constitutes the filling material itself, and possibly can, during preparation, be admixed with a further substance.

The filling material, by virtue of the step of preparation and solidification to which it is subjected, is porous.

As stated above, according to one version of the present invention the containment body 1 is porous, since it has pores 6.

In one version of the invention, the pores 6 can be interconnected with each other and/or uniformly distributed through the volume of the containment body 1 and, thus, through its base portion 2 and its side walls 3.

As is better described hereinbelow, the pores 6 place the at least one cavity 4 in mutual communication with the outside of the containment body 1.

Therefore, if the pharmaceutical or medical substance is placed inside the at least one cavity 4 of the containment body 1 - in the filling material - the pores 6 facilitate the release thereof also through the containment body 1 itself.

According to one aspect of the present invention, the size of the pores 6 is such to prevent, during use, bone regrowth inside the containment body 1 and, hence, inside the temporary and/or disposable spacer device which comprises it.

Such configuration of the pores 6, therefore, facilitates the subsequent removal of the spacer device from the treated bone seat or joint seat, once its curative function has been completed.

By way of example, the pores 6 can have, in one version, average dimensions of less than 100 micron.

According to a further version of the present invention, the containment body 1 can have a plurality of through openings, not illustrated in the figures, adapted to place the interior of the at least one cavity 4 in communication with the outside of the containment body 1 itself.

Analogous to that described above, in one version of the invention, such through openings can have size such to not allow bone growth through the portions of the containment body 1.

The through openings can have a circular section or any other geometry suitable for such purpose.

According to another version of the present invention, the containment body 1 can be porous, and thus comprise pores 6, and also have a plurality of through openings, with the same objectives described for the preceding versions.

According to one version of the present invention, the containment body 1 can comprise at least one separating wall 7, represented in the enclosed figures with a discontinuous dashed line, positioned inside the at least one cavity 4 for the purpose of dividing the latter into multiple portions separated from each other.

The at least one separating wall 7 identifies spaces 4', 4" inside the at least one cavity 4 that are separated from each other, for housing respective filling materials that are possibly different from each other.

Such possibility is particularly advantageous if a surgeon intends to use different types of pharmaceutical or medical substances for treating specific portions of the bone seat or joint seat.

The at least one separating wall 7 is projected upward from the base portion 2 and/or from at least one of the side walls 3.

In one version, the containment body 1 can comprise at least one separating wall 7 which identifies, inside the at least one cavity 4, two portions 4', 4" that are separated from each other (see figure 1).

In a further version, the at least one separating wall 7 identifies, inside the at least one cavity 4, three portions 4', 4", 4‴ that are separated from each other (see figure 2).

Further versions are possible comprising a greater number of separating walls 7, possibly differently configured with respect to that illustrated in the enclosed figures, without departing from the protective scope of the present invention.

For such purpose, according to a further version of the present invention, the containment body 1 comprises a plurality of walls 7 interconnected with each other to constitute a substantially trabecular structure, comprising a plurality of cavities.

Such substantially trabecular structure can be filled or impregnated with at least one pharmaceutical or medical substance.

With reference to such version, and as stated above, the containment body 1 in one version of the invention is porous and therefore has a plurality of pores 6 adapted to place the cavities present in the trabecular structure in communication with the outside of the containment body 1.

According to a further version of the present invention, the containment body 1 can have a plurality of openings passing through the side walls 3 and/or the base portion 2, thus placing the cavities present in the trabecular structure in communication with the outside of the containment body 1 itself.

According to another version, the containment body 1 can be porous and have a plurality of through openings adapted to place the cavities of the trabecular structure in communication with the outside of the containment body 1.

According to one version of the invention, the at least one wall 7 is made of the same material with which the containment body 1 is made.

According to one aspect of the present invention, the containment body 1 is associable with a closing body 9, for closing the access opening 5 to the at least one cavity 4 (figures 3-5).

In one version of the invention, the closing body 9 has a shape substantially corresponding to that of the opening 5.

With the containment body 1 and the closing body 9 associated with each other, in practice, one obtains a closed casing that is externally configured to reproduce, in a substantially complementary manner, the shape of the bone seat or joint seat to be treated or part thereof.

As stated above, inside such closed casing, at least one cavity 4 is present which can be filled with the above-described filling.

The closing body 9 has a first surface 10 and a second surface 11, opposite each other.

Analogous to that described for the containment body 1, also the closing body 9 can be porous.

More in detail, the closing body 9 can comprise a plurality of pores 12.

In one version of the invention, the pores 12 are interconnected with each other, adapted to place the first surface 10 in mutual communication with the second surface 11, hence the cavity 4 with the outside of the casing.

The pores 12 of the closing body 9 substantially have the same characteristics as the above-described pores 6 of the containment body 1.

The pores 12 are present in a substantially uniform manner in the entire closing body 9; therefore, the release of at least one pharmaceutical or medical substance can also occur through the closing body 9 and, more precisely, through the pores 12 present therein.

According to a further version of the present invention, the closing body 9 can have at least one through opening, with limited size, through the first surface 10 and the second surface 11, not illustrated in the figures.

Actually, the at least one through opening determines a path adapted to place the first surface 10 in communication with the second surface 11.

According to another version of the present invention, the closing body 9 can be porous and have at least one through opening, described above.

In figure 4, a further embodiment of a containment body according to the present invention is illustrated, indicated with the reference number 100, provided for making a temporary and/or disposable spacer device for the knee joint.

More in detail, the containment body 100 is provided for making a spacer device constrainable to the femoral portion of the knee joint.

Hereinbelow, the same reference numbers will be used for indicating the same components described in the preceding embodiment, increased by 100.

The containment body 100 overall differs from the preceding embodiment only due to the shape thereof.

In particular, the containment body 100 comprises a base portion 102 from which side walls 103 perimetrically extend, configured so as to reproduce the femoral bone end of the knee, to which the containment body 100 must be constrained. In particular, the base surface 102 corresponds with the surface of the femoral bone that articulates on the tibial bone end.

The containment body 100 has at least one cavity 104, for housing at least one filling material, preferably of solidifiable type.

Analogous to that described for the preceding embodiment, the containment body 100 can have an opening 105, delimited by the side walls 103, in particular by their outer perimeter edges, for the access to the at least one cavity 104.

According to one version of the present invention, the containment body 100 can comprise at least one separating wall 107, illustrated with a discontinuous dashed line, in order to delimit, inside the at least one cavity 104, at least two portions 104', 104".

The at least one separating wall 107 can have a different shape from that illustrated in the enclosed figures, in order to delimit a greater number of spaces in the at least one cavity 104, without any limitation.

According to one version, the containment body 100 can comprise a plurality of walls 107 configured so as to define a substantially trabecular structure inside the containment body 100 itself.

A substantially trabecular structure thus defined comprises a plurality of cavities 104 which can be filled or impregnated with at least one filling material.

The containment body 100 is associable with a closing body 109, adapted to close the opening 105.

The closing body 109 corresponds to the surface to be constrained to the femoral end of the knee, so as to recreate, together with the containment body 100, the complete femoral end of the knee and allow the articulation with a tibial component or with the tibial bone end of the knee.

During use, the containment body 100 and the closing body 109, coupled together, constitute a closed casing provided with at least one internal cavity 104 in which at least one filling material, of solidifiable type, is housable.

The containment body 100, along with the closing body 109, can be porous and more in detail can have a plurality of pores, respectively indicated with 106 and 112, adapted to place the at least one internal cavity 104 in communication with the outside of the containment body 100 and of the closing body 109.

According to one aspect of the present invention, the containment body 100 and/or the closing body 109 can have a connection element 120 that is extended from the closing body 109, moving away therefrom.

With reference to the embodiment illustrated in figure 4, the connection element 120 extends from the closing body 109 and has a substantially elongated shape, similar to a pin, and is adapted to be introduced and housed in a seat obtainable at the femoral end to be treated in proximity to the knee joint.

In general, the connection element 120 can in any case have any other shape adapted to facilitate the connection of the closing body 109 to a bone seat or end, without any limitation.

In figures 5 and 6, a further embodiment of a containment body according to the present invention is illustrated, indicated overall with the number 200.

Hereinbelow, the same components corresponding to those of the previously-described embodiments will be indicated with the same reference numbers, increased by 100.

The containment body 200 overall differs from the preceding embodiments due to its shape.

More precisely, the containment body 200 is shaped for making a temporary and/or disposable spacer device for the articulation of the shoulder or hip.

The containment body 200 has a base portion 202 from which side walls 203 perimetrically extend, delimiting at least one cavity 204.

The at least one cavity 204 is accessible from an opening 205 delimited by the side walls 203, in particular by their external perimeter edges.

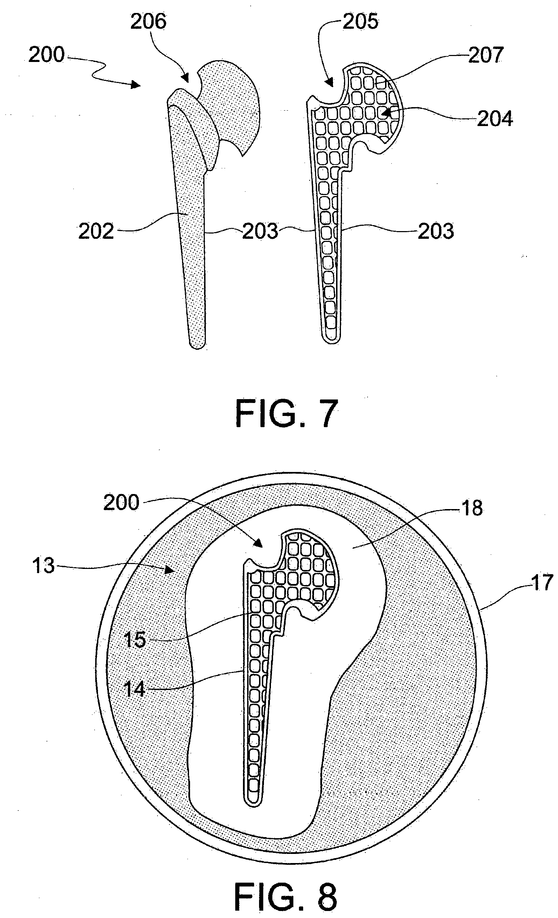

The containment body 200 can comprise at least one wall 207 (see figures 7 and 8) adapted to delimit at least one cavity 204 inside the containment body 200.

The containment body 200 can also comprise a plurality of walls 207 configured so as to define a substantially trabecular structure, comprising a plurality of cavities 204.

Since the containment body 200 can be porous and/or comprise at least one through opening through the side walls 203 and/or the base portion 202, the at least one cavity 204 is in communication with the outside of the containment body 200.

As described for the preceding embodiments, the containment body 200 is associable with a closing body 209, for closing the access opening 205 to the at least one cavity 204.

During use, the containment body 200 and the closing body 209 associated with each other constitute a closed casing.

For such purpose, it is observed that if the spacer device to be made has a plane of symmetry, the containment body and the closing body can be shaped specular to each other.

By way of example, the version of the spacer device illustrated in figure 5 has a plane of symmetry, overall indicated with VI-VI.

According to such version, therefore, the closing body 209 and the containment body 200 are shaped specular to each other.

If the closing body 9, 109, 209 is provided, constraining means (not illustrated in the figures) can be present, in a manner so as to constrain such closing body 9, 109, 209 to the respective containment body 1, 100, 200, once the filling of the at least one cavity 4, 104, 204 with the filling material has been completed.

Such constraining means can be present in the outer peripheral edges of the containment body 1, 100, 200 and/or in the outer peripheral edges of the closing body 9, 109, 209.

The constraining means can for example comprise complementary edges, adapted to be inserted in the corresponding edges of the opposite body and be retained thereby.

Alternatively, the constraining means can be snap or bayonet constraining means, so as to stably connect the containment body 1, 100, 200 together with the closing body 9, 109, 209.

According to one aspect of the present invention, in order to make device according to the present invention, the shape of the spacer device to be made is first established, then a hollow figure is generated corresponding to that of the spacer device to be made, then a plane is identified, possibly a plane of symmetry or line, of the spacer device, so as to obtain a containment body, and possibly a closing body, corresponding to and suitable for the spacer device to be made.

In such a manner, the geometry or shape of the containment body and of the possible closing body is established, and potentially any geometry of a spacer device or implantable device for the human body can be reproduced.

The containment body 1, 100, 200, along with the closing body 9, 109, 209 when present, can be made by means of a mold forming process.

By way of a non-limiting example, such process can be a sintering process.

The latter, in brief, comprises an initial step of forming a product starting from a powder material or a mixture of powder materials which, initially, are pressed and formed inside a mold.

Subsequently, the pressed component thus formed, also known as green component, is subjected to the sintering step by means of which the single granules are at least partially melted together, thus determining a compact finished product.

The powder material to be sintered generally is mixed with solvents, glues or binders that facilitate the initial forming step thereof (generating a partial gluing of the powders), which are subsequently dispersed during the subsequent process steps.

By way of example, the powder material can have a grain size comprised between 1 and 1000 micron.

The sintering step occurs at high temperature, by means of dry heat or by means of vapor, leading to an at least partial superficial thermal melting. High pressure, solvent or binder and heat can also be used simultaneously.

Such powder material can for example comprise materials such as thermoplastic or thermosetting plastics, metals, ceramics, composite materials and generally materials that are biocompatible and implantable in the human body, hence without causing a rejection by the tissues with which they are placed in contact.

The powder material can belong to other material types with respect to those indicated above, as long as they are of biocompatible type and implantable in the human body without any limitation.

By means of the aforesaid sintering process, it is possible to obtain a containment body 1, 100, 200 and/or a closing body 9, 109, 209 that is compact or porous, in the latter case comprising a plurality of pores, possibly intercommunicating with each other, such to place the interior of the at least one cavity 4, 104, 204 in communication with the outside environment.

According to a further version, the containment body 1, 100, 200 and the closing body 9, 109, 209, when provided, can be made by means of a process of injection molding, e.g. in a press, a thermoplastic resin of biocompatible type and implantable in the human body.

In order to confer an open porosity to the containment body 1, 100, 200 and/or to the closing body 9, 109, 209, additives can be admixed with the resin itself, such as inorganic salts or sodium chloride.

As a function of the level of porosity that one intends to confer to the containment body 1, 100, 200 and/or to the closing body 9, 109, 209, the additives can be added in variable quantities comprised between 1% and 99%, while the remaining part is constituted by the resin itself; as can be perceived, the greater the quantity of additive added to the resin, the greater the porosity of the obtained manufactured product.

Such porosity is manifested by subjecting the manufactured item to successive baths in suitable solvents, such as water or vapor, removing the additives that therefore leave voids or cavities in the manufactured item itself.

The number of the cavities left by the additives, and the size thereof, correspond to the size and quantities of the additive itself.

In a further version of the invention, the additives can be solid, liquid, gaseous or mixtures thereof, of organic or inorganic type.

In this case, the manufactured products can be made in series, only constrained to the figure or shape that one wishes to obtain.

According to a further version, the containment body 1, 100, 200, along with the closing body 9, 109, 209 when present, can be made by means of an additive process, such as for example a three-dimensional printing process of a biocompatible material that is implantable in the human body, of the previously described type or preferably of acrylic nature.

By means of the three-dimensional printing process, it is possible to obtain a containment body 1, 100, 200, and possibly a closing body 9, 109, 209, of porous type or having a honeycomb structure and hence substantially trabecular.

Indeed, the three-dimensional printing process allows attaining possible walls 7, 107, 207 inside the cavity 4, 104, 204 directly during the step of forming/printing the containment body 1, 100, 200, thus limiting the overall obtainment times.

According to one version of the present invention, the walls 7, 107, 207 that can be made through the three-dimensional printing process can be configured so as to define a substantially trabecular structure for the containment body 1, 100, 200.

According to one version of the present invention, in the three-dimensional printing process, for example, provision can be made for the use of wire materials constituted by thermoplastic resins such as PMMA, PE, PEEK etcetera.

Nevertheless, it is possible to use materials different from those described above, as long as they are of biocompatible type and implantable in the human body, without departing from the protective scope of the present invention.

The honeycomb structure or trabecular structure, once attained, is filled with a filling material which, by solidifying or hardening, determines the obtainment a spacer device or device to be implanted, integrally made with the containment body and the possible closing body.

In this manner, it is possible to obtain custom or personalized/personalizable manufactured items for each single patient.

The custom manufactured items can thus be made of plastic or metal materials, e.g. by means of laser sintering of metal powder etc., also with the obtainment of complex structures that cannot be made via injection press. For example, it is possible to make containment bodies of at least one pharmaceutical or medical substance to be released to their exterior, and successive spacers for any region of the human body, or a prosthesis for the cranium or prosthesis for the chest (e.g. from the TAC data).

As stated above, a temporary and/or disposable spacer device, implantable in the human body for treating an infected bone seat or joint seat, comprises a containment body, object of the present invention.

The containment body and the possible closing body, if present, must correspond with the external geometry of the spacer to be made, since the outer portion of such spacer device - and hence its shape and size - correspond with those of the containment body and/or closing body that determine the outer portion and surface thereof.

According to one version, the hardening of the filling material, in fact, unites the containment body and possible closing body in a single, solid and rigid structure, to be implanted in the human body.

Such temporary and/or disposable spacer device, hereinbelow spacer device, is overall indicated with the reference number 13.

The temporary and disposable spacer device 13 comprises an outer portion 14, configured in a substantially complementary manner to the shape of the bone seat or joint seat with which it must be associated, or with a part thereof, and an inner portion 15.

During use, the outer portion 14 and the inner portion 15 are constrained to each other.

According to one version, the outer portion comprises a containment body 1, 100, 200.

More precisely, the outer portion 14 corresponds with the containment body 1, 100, 200.

According to another version, the outer portion 14 comprises a containment body 1, 100, 200 and a closing body 9, 109, 209, associable with each other to form a hollow casing.

According to one version of the present invention, the inner portion 15 of the spacer device 13 can comprise at least one filling material of biologically compatible type, introduced inside the cavity 4, 104, 204 of the containment body 1, 100, 200.

According to an example that does not form part of the present invention, the filling material can be a liquid or a fluid, more or less viscous, capable of being eluted through the pores 6, 106, 12, 112 respectively present in the containment body 6, 106 and in the closing body 9, 109.

The filling material can comprise at least one pharmaceutical or medical substance to be released through the outer portion 14 of the spacer device 13 which is placed in contact with the bone tissue to be treated.

Preferably, the aforesaid liquid or fluid filling material is usable in a spacer device 13 comprising a containment body 1, 100, 200 with sufficiently thick walls (e.g. 20 mm), such to confer high mechanical strength to the spacer device 13 itself.

According to a further version of the present invention, the filling material can be a fluid of hardening or solidifiable type.

In such case, by way of example, the filling material can be bone cement.

According to one version, the filling material is prepared by the surgeon, who admixes at least one pharmaceutical or medical substance with the components of the filling material.

The use of a filling material of hardening type can be provided independent of the thickness of the walls of the containment body 1, 100, 200.

According to another version of the present invention, the spacer device 13 can comprise, in the inner portion 15, at least one reinforcing core 16 (illustrated for example in figure 6) for the spacer device 13 itself.

According to a further version of the present invention, the inner portion of the spacer device 13 can comprise a plurality of walls 7, 107, 207 configured in a manner such to define a substantially trabecular structure, which can be filled or impregnated with at least one pharmaceutical or medical substance.

The at least one wall 7, 107, 207 can constitute a single body with the containment body 1, 100, 200; in such version, the assembly given by the at least one wall 7, 107, 207 and the containment body 1, 100, 200 and possibly the closing body 9, 109, 209 therefore forms the structure of the spacer device 13.

In figure 8, a section of a spacer device 13 is depicted and, more in detail, a containment body 200 is depicted, in abutment against a Petri dish 17.

According to such version, the outer portion 14 is porous and the inner portion 15 comprises at least one pharmaceutical or medical substance.

On the Petri dish, a bacteria culture 18 is present, illustrated as a ring-like area.

As stated above, the outer portion 14 of the spacer device 13 is porous and therefore the at least one pharmaceutical or medical substance present in the inner portion 15 can exit outward through the pores of the outer portion 14, thus determining a region 19 of bacteria growth inhibition in the immediate vicinity of the spacer device 13 itself.

According to a further aspect of the present invention, the temporary and disposable spacer device 13 can comprise at least one specific diagnostic or measurement device, not illustrated in the figures, housed inside the containment body 1, 100, 200 or, specifically, inside the at least one cavity 4, 104, 204.

By way of example, the spacer device 13 could comprise a biomedical/biological micro-electromechanical system, such as a bio-sensor, capable of carrying out detections of chemical-physical type. One such bio-sensor, which corresponds to a chip, could comprise a miniaturized circuit in turn comprising an accelerometer and/or a thermometer and/or a load cell and/or sensors adapted to detect further physical entities of different type.

The chip to be associated inside a spacer device 13 can be selected as a function of specific use needs and of the type of detections to be carried out.

By associating such chip with the spacer device 13, it is therefore possible to detect the use conditions of the spacer device 13 itself, with reference for example to the accelerations and/or to the static or dynamic loads to which it is subjected, to the temperature of the bone seat or joint seat in which it is implanted, etcetera.

According to a further example, the bio-sensor could comprise an integrated interface for transferring the detected data.

By way of example, the bio-sensor could comprise data transmission means for allowing the real-time detection of the use conditions of the spacer device 13 with which it is associated.

Hereinbelow, a method is described for making a temporary and disposable spacer device according to the present invention.

The aforesaid method, initially, provides for supplying a containment body 1, 100, 200, shaped in a manner such to be able to be inserted in and/or associated with the bone seat or joint seat to be treated or with a part thereof.

Subsequently, a filling material is introduced through the opening 5, 105, 205, inside the cavity 4, 104, 204 present in the containment body 1, 100, 200.

As stated above, the usable filling material is of solidifiable type, such as bone cement.

Possibly, during the filling step or in the preceding instants, at least one bio-sensor can be introduced inside the containment body 1, 100, 200 for the detection of the use conditions of the spacer device 13.

If provided, the containment body 1, 100, 200 is associable with a closing body 9, 109, 209, adapted to close the opening 5, 105, 205 and hence the cavity 4, 104, 204.

If provided, the at least one bio-sensor is stably constrained inside the containment body 1, 100, 200 following the hardening or solidification of the filling material that surrounds it or, if present, by means of the closing body 9, 109, 209 possibly associated with the containment body 1, 100, 200 itself, which prevents the exit of the bio-sensor from the spacer device 13.

When provided, the closing body 9, 109, 209 is constrained to the containment body 1, 100, 200 after having filled the at least one cavity 4, 104, 204 with the filling material. In the version in which the filling material is solidifiable, during such solidification, the filling material forms a single body, adapted to be implanted in the human body, together with the containment body 1, 100, 200 and with the closing body 9, 109, 209.

The spacer device 13 is ready for its use following the hardening of the filling material present inside the at least one cavity 4, 104, 204.

According to one version of the present method, a step can be provided for inserting at least one reinforcing core 16 inside the at least one cavity 4, 104, 204 before or after the introduction of the filling material in the cavity 4, 104, 204 itself.

If constraining means are provided, once the filling of the at least one cavity 4, 104, 204 with the filling material is completed, the closing body 9, 109, 209 is constrained to the respective containment body 1, 100, 200.

In accordance with such version, the inner portion 15 comprises at least one filling material and at least one reinforcing core 16.

As stated above, the containment body 1, 100, 200 and the closing body 9, 109, 209 associable therewith are porous.

Therefore, the at least one pharmaceutical or medical substance comprised in the filling material or possibly that contained or impregnated in the containment body 1, 100, 200 and/or in the closing body 9, 109, 209 if present, can be gradually released, and in a substantially uniform manner, through the entire outer surface of the spacer device 13.

According to a further version, the filling material may lack pharmaceutical or medical substances; at least one such substance can be directly impregnated in the porosity of the containment body 1, 100, 200 and/or, if provided, in the closing body 9, 109, 209, and/or in the filling material.

The containment body 1, 100, 200, possibly associated with the closing body 9, 109, 209, is therefore an integral part of the spacer device 13 and is thus implanted or adapted to be implanted in the human body.

According to such version, the filling material can be a liquid or a fluid of solidifiable type or of non-solidifiable type.

With reference to the latter case, the liquid or fluid can be more or less viscous, as a function of the duration of the overall treatment period of the infected site, and can comprise at least one pharmaceutical or medical substance, capable of exiting out of the pores 6, 106, 12, 112 present in the outer portion 14 of the spacer device 13. In one version of the invention, the liquid or fluid can have a pasty or gelatinous consistency, or in any case be able to remain in the insertion site, for example without percolating.

The spacer device 13 according to the present invention is capable of ensuring a gradual release of at least one pharmaceutical substance, even for prolonged time periods.

In the inner surface of the containment body and/or of the possible closing body, stalks, eversions or undercuts (not illustrated) can be present with the function of facilitating the anchoring of the filling material to the walls of the containment body or of the closing body themselves.

The given definition of "porous" element, present in the present discussion, can be substituted by "semi-permeable", without departing from the protective scope of the present invention.

The invention thus conceived is susceptible of numerous modifications and variants, all falling within the scope of the inventive concept.

The characteristics present for one version or embodiment can be combined with the characteristics of another version or embodiment, without departing from the protective scope of the present invention.

In practice, the materials used, as well as the contingent shapes and sizes, can be of any type in accordance with the requirements, without departing from the protective scope of the invention, which is defined by the following claims.

## Claims

1. Containment body (1, 100, 200) configured for making a temporary and/or disposable spacer device (13) to be implanted in the human body, for treating an infected bone seat or a joint seat inside the human body, said containment body (1, 100, 200) comprising a base portion (2, 102, 202), side walls (3, 103, 203) that extend from said base portion (2, 102, 202), said base portion (2, 102, 202) and said side walls (3, 103, 203) delimiting inside them at least one cavity (4, 104, 204), and comprising at least one opening (5, 105, 205) delimited by said side walls (3, 103, 203) for accessing to said at least one cavity (4, 104, 204), wherein said base portion (2, 102, 202) and said side walls (3, 103, 203) are made from biologically compatible material, of the type implantable inside the human body, wherein said base portion (2, 102, 202) and/or at least one of said side walls (3, 103, 203) have a plurality of pores (6, 106, 206) interconnected with one another and/or at least one through opening, configured so as to place said at least one cavity (4, 104, 204) in communication with the outside of said containment body (1, 100, 200), wherein said at least one cavity (4, 104, 204) is configured for being filled with a filling material that is a solidifiable fluid, and wherein said base portion (2, 102, 202) and said side walls (3, 103, 203) have a thickness between 0.5 mm and 20 mm.

2. Containment body (1, 100, 200) according to claim 1, wherein said side walls (3, 103, 203) extend perimetrically from said base portion (2, 102, 202) and/or wherein said side walls (3, 103, 203) are continuous of one another and/or wherein said base portion (2, 102, 202) and said side walls (3, 103, 203) are shaped so as to substantially match the bone seat or the joint seat or a part thereof in which said containment body (1, 100, 200) can be implanted in use.

3. Containment body (1, 100, 200) according to claim 1 or 2, wherein said at least one opening (5, 105, 205) is delimited by an outer peripheral edge of said side walls (3, 103, 203), for accessing to said at least one cavity (4, 104, 204) and/or wherein said containment body comprises a closing body (9, 109, 209) suitable for closing said at least one cavity (4, 104, 204).

4. Containment body (1, 100, 200) according to claim 3, comprising constraining means for constraining said closing body (9, 109, 209) with said containment body (1, 100, 200).

5. Containment body (1, 100, 200) according to any one of the previous claims, comprising at least one separating wall (7, 107, 207) inside said at least one cavity (4, 104, 204), said at least one separating wall (7, 107, 207) projecting upwards from said base portion (2, 102, 202) and/or from at least one of said side walls (3, 103, 203), said at least one separating wall (7, 107, 207) defining at least two portions (4', 4", 104', 104'') inside the volume of said at least one cavity (4, 104, 204).

6. Containment body (1, 100, 200) according to claim 5, comprising a plurality of said separating walls (7, 107, 207) configured so as to define a substantially trabecular structure inside said containment body (1, 100, 200).

7. Containment body (1, 100, 200) according to any previous claim, wherein the thickness of said base portion (2, 102, 202) and of said side walls (3, 103, 203) is between 0.5 mm a 4 mm.

8. Temporary and/or disposable spacer device (13), implantable in the human body for the treatment of a bone seat or of a joint seat, comprising an outer portion (14), having a shape substantially matching the shape of the bone seat or of the joint seat to be treated or part thereof to which said spacer device (13) must be constrained, and an inner portion (15), wherein said outer portion (14) comprises a containment body (1, 100, 200) according to any one of claims 1 to 7, wherein the at least one cavity (4, 104, 204) is filled with said inner portion (15) which includes a filling material that is a solidified fluid, and wherein said inner portion (15) comprises bone cement and at least one pharmaceutical or medical substance.

9. Spacer device (13) according to claim 8, wherein said inner portion (15) is contained in said containment body (1, 100, 200).

10. Spacer device according to claim 8 or 9, wherein said outer portion (14) further comprises a closing body (9, 109, 209) for closing said at least one cavity (4, 104, 204) of said containment body (1, 100, 200), said closing body (9, 109, 209) having a first surface (10, 110), in use outside said at least one cavity (4, 104, 204), and a second surface (11, 111) opposite said first surface (10, 110), in use inside said at least one cavity (4, 104, 204) and/or wherein said containment body (1, 100, 200) and/or said closing body (9, 109, 209) comprises at least one pharmaceutical or medical substance.

11. Spacer device according to claim 10, wherein said closing body (9, 109, 209) is porous, comprising a plurality of pores (12, 112, 212) interconnected with each other, and/or a plurality of pores (12, 112, 212) and/or at least one through opening, configured so as to place said first surface (10, 110) in communication with said second surface (11, 111).

12. Spacer device according to claim 8, wherein said inner portion (15) comprises at least one reinforcing core (16).

13. Spacer device according to any one of claims 8 to 12, comprising at least one bio-sensor positioned inside said containment body (1, 100, 200), for detecting at least one chemical-physical magnitude.

14. Method for making a temporary and/or disposable spacer device implantable in the human body for the treatment of an infected bone seat or of a joint seat according to any one of claims 8 to 13, comprising the steps of:
providing at least one containment body (1, 100, 200) shaped like the bone seat or the joint seat to be treated or part thereof and provided with at least one cavity (4, 104, 204), so as to constitute an outer portion (14) of said spacer device,
providing a filling material,
inserting or impregnating the filling material in said at least one cavity (4, 104, 204),
setting or shaping said filling material, so as to constitute an inner portion (15) of said spacer device.

15. Method according to claim 14, comprising the steps of:
providing a closing body (9, 109, 209),
associating said closing body (9, 109, 209) with said containment body (1, 100, 200) so as to define a casing inside which said inner portion (15) is, and/or
constraining said closing body (9, 109, 209) to said containment body (1, 100, 200) through constraining means.

16. Method according to claim 14, wherein said step of constituting said inner portion (15) foresees a step of removing a excess of said filling material that protrudes from said containment body (1, 100, 200) or comprising a step of inserting a reinforcing core (16) inside said inner portion (15).

## Patentansprüche

1. Aufnahmekörper (1, 100, 200), konfiguriert zur Herstellung einer temporären und/oder entsorgbaren Abstandsvorrichtung (13), die zur Behandlung eines infizierten Knochensitzes oder eines Gelenksitzes innerhalb des menschlichen Körpers in den menschlichen Körper zu implantieren ist, der besagte Aufnahmekörper (1, 100, 200) umfassend einen Basisabschnitt (2, 102, 202), Seitenwände (3, 103, 203), die sich von dem besagten Basisabschnitt (2, 102, 202) erstrecken, wobei der besagte Basisabschnitt (2, 102, 202) und die besagten Seitenwände (3, 103, 203) in ihrem Inneren mindestens eine Kavität (4, 104, 204) begrenzen, und umfassend mindestens eine Öffnung (5, 105, 205), die durch die besagten Seitenwände (3, 103, 203) für den Zugang zu der besagten mindestens einen Kavität (4, 104, 204) begrenzt wird, worin der besagte Basisabschnitt (2, 102, 202) und die besagten Seitenwände (3, 103, 203) aus biologisch kompatiblem Material des Typs, der in den menschlichen Körper implantierbar ist, hergestellt sind, worin der besagte Basisabschnitt (2, 102, 202) und/oder mindestens eine der besagten Seitenwände (3, 103, 203) eine Vielzahl von Poren (6, 106, 206), die miteinander verbunden sind, und/oder mindestens eine Durchgangsöffnung, die konfiguriert ist, um die besagte mindestens eine Kavität (4, 104, 204) in Verbindung mit der Außenseite des besagten Aufnahmekörpers (1, 100, 200) anzuordnen, aufweisen, worin die besagte mindestens eine Kavität (4, 104, 204) konfiguriert ist, um mit einem Füllmaterial gefüllt zu werden, das ein verfestigbares Fluid ist, und worin der besagte Basisabschnitt (2, 102, 202) und die besagten Seitenwände (3, 103, 203) eine Dicke zwischen 0,5 mm und 20 mm aufweisen.

2. Aufnahmekörper (1, 100, 200) nach Anspruch 1, worin sich die besagten Seitenwände (3, 103, 203) perimetrisch von dem besagten Basisabschnitt (2, 102, 202) erstrecken und/oder worin die besagten Seitenwände (3, 103, 203) durchgehend miteinander verbunden sind und/oder worin der besagte Basisabschnitt (2, 102, 202) und die besagten Seitenwände (3, 103, 203) so geformt sind, dass sie im Wesentlichen mit dem Knochensitz oder dem Gelenksitz oder einem Teil davon, in den der besagte Aufnahmekörper (1, 100, 200) bei Verwendung implantiert werden kann, übereinstimmen.

3. Aufnahmekörper (1, 100, 200) nach Anspruch 1 oder 2, worin die besagte mindestens eine Öffnung (5, 105, 205) durch eine äußere Umfangskante der besagten Seitenwände (3, 103, 203) begrenzt ist, um Zugang zu der besagten mindestens einen Kavität (4, 104, 204) zu erhalten, und/oder worin der besagte Aufnahmekörper einen Verschlusskörper (9, 109, 209), der zum Verschließen der besagten mindestens einen Kavität (4, 104, 204) geeignet ist, umfasst.

4. Aufnahmekörper (1, 100, 200) nach Anspruch 3, umfassend Befestigungsmittel zum Befestigen des besagten Verschlusskörpers (9, 109, 209) an dem besagten Aufnahmekörper (1, 100, 200).

5. Aufnahmekörper (1, 100, 200) nach irgendeinem der vorangegangenen Ansprüche, umfassend mindestens eine Trennwand (7, 107, 207) innerhalb der besagten mindestens einen Kavität (4, 104, 204), wobei die besagte mindestens eine Trennwand (7, 107, 207) von dem besagten Basisabschnitt (2, 102, 202) und/oder von mindestens einer der besagten Seitenwände (3, 103, 203) nach oben vorsteht, wobei die besagte mindestens eine Trennwand (7, 107, 207) mindestens zwei Abschnitte (4', 4", 104', 104") innerhalb des Volumens der besagten mindestens einen Kavität (4, 104, 204) definiert.

6. Aufnahmekörper (1, 100, 200) nach Anspruch 5, umfassend eine Vielzahl der besagten Trennwände (7, 107, 207), die so konfiguriert sind, dass sie eine im Wesentlichen trabekuläre Struktur innerhalb des besagten Aufnahmekörpers (1, 100, 200) definieren.

7. Aufnahmekörper (1, 100, 200) nach irgendeinem vorangegangenen Anspruch, worin die Dicke des besagten Basisabschnitts (2, 102, 202) und der besagten Seitenwände (3, 103, 203) zwischen 0,5 mm und 4 mm liegt.

8. Temporäre und/oder entsorgbare Abstandsvorrichtung (13), die in den menschlichen Körper zur Behandlung eines Knochensitzes oder eines Gelenksitzes implantierbar ist, mit einem äußeren Abschnitt (14), der eine Form aufweist, die im Wesentlichen der Form des zu behandelnden Knochensitzes oder Gelenksitzes oder eines Teils davon entspricht, an dem die besagte Abstandsvorrichtung (13) befestigt werden muss, und einem inneren Abschnitt (15), worin der besagte äußere Abschnitt (14) einen Aufnahmekörper (1, 100, 200) nach irgendeinem der Ansprüche 1 bis 7 umfasst, worin die besagte mindestens eine Kavität (4, 104, 204) mit dem besagten inneren Abschnitt (15) gefüllt ist, der ein Füllmaterial enthält, das eine verfestigte Flüssigkeit ist, und worin der besagte innere Abschnitt (15) Knochenzement und mindestens eine pharmazeutische oder medizinische Substanz umfasst.

9. Abstandsvorrichtung (13) nach Anspruch 8, worin der besagte innere Abschnitt (15) in dem besagten Aufnahmekörper (1, 100, 200) enthalten ist.

10. Abstandsvorrichtung nach Anspruch 8 oder 9, wobei der besagte äußere Abschnitt (14) ferner einen Verschlusskörper (9, 109, 209) zum Verschließen der besagten mindestens einen Kavität (4, 104, 204) des besagten Aufnahmekörpers (1, 100, 200) umfasst, wobei der besagte Verschlusskörper (9, 109, 209) eine erste Oberfläche (10, 110) aufweist, die bei Verwendung außerhalb der besagten mindestens einen Kavität (4, 104, 204) liegt, und eine zweite Oberfläche (11, 111) gegenüberliegend der besagten ersten Oberfläche (10, 110), die bei Verwendung innerhalb der besagten mindestens einen Kavität (4, 104, 204) liegt, und/oder worin der besagte Aufnahmekörper (1, 100, 200) und/oder der besagte Verschlusskörper (9, 109, 209) mindestens eine pharmazeutische oder medizinische Substanz umfasst.

11. Abstandsvorrichtung nach Anspruch 10, worin der besagte Verschlusskörper (9, 109, 209) porös ist, umfassend eine Vielzahl von Poren (12, 112, 212), die miteinander verbunden sind, und/oder eine Vielzahl von Poren (12, 112, 212) und/oder mindestens eine Durchgangsöffnung, die konfiguriert ist, um die besagte erste Oberfläche (10, 110) in Verbindung mit der besagten zweiten Oberfläche (11, 111) anzuordnen.

12. Abstandsvorrichtung nach Anspruch 8, worin der besagte innere Abschnitt (15) mindestens einen Verstärkungskern (16) umfasst.

13. Abstandsvorrichtung nach irgendeinem der Ansprüche 8 bis 12, umfassend mindestens einen Biosensor, der im Inneren des besagten Aufnahmekörpers (1, 100, 200) angeordnet ist, um mindestens eine chemischphysikalische Größe zu erfassen.

14. Verfahren zur Herstellung einer temporären und/oder entsorgbaren Abstandsvorrichtung, die in den menschlichen Körper zur Behandlung eines infizierten Knochensitzes oder Gelenksitzes nach irgendeinem der Ansprüche 8 bis 13 implantierbar ist, umfassend die Schritte des:
Bereitstellens mindestens eines Aufnahmekörpers (1, 100, 200), der wie der zu behandelnde Knochensitz oder Gelenksitz oder eines Teils davon geformt und mit mindestens einer Kavität (4, 104, 204) versehen ist, um einen äußeren Abschnitt (14) der besagten Abstandsvorrichtung zu bilden,
Bereitstellens eines Füllmaterials,
Einsetzens oder Imprägnieren des Füllmaterials in die besagte mindestens eine Kavität (4, 104, 204),
Einrichtens oder Formens des besagten Füllmaterials, um einen inneren Abschnitt (15) der besagten Abstandsvorrichtung zu bilden.

15. Verfahren nach Anspruch 14, umfassend die Schritte des:
Bereitstellens eines Verschlusskörpers (9, 109, 209),
Verbindens des besagten Verschlusskörpers (9, 109, 209) mit dem besagten Aufnahmekörper (1, 100, 200), um ein Gehäuse zu definieren, in dem sich der besagte innere Abschnitt (15) befindet, und/oder
Befestigens des besagten Verschlusskörpers (9, 109, 209) an dem besagten Aufnahmekörper (1, 100, 200) durch Befestigungsmittel.

16. Verfahren nach Anspruch 14, worin der besagte Schritt des Bildens des besagten inneren Abschnitts (15) einen Schritt des Entfernens eines Überschusses des besagten Füllmaterials, das aus dem besagten Aufnahmekörper (1, 100, 200) herausragt, vorsieht oder einen Schritt des Einsetzens eines Verstärkungskerns (16) in den besagten inneren Abschnitt (15) umfasst.

## Revendications

1. Corps de confinement (1, 100, 200) configuré pour fabriquer un dispositif d'espacement temporaire et/ou jetable (13) à implanter dans le corps humain, pour traiter un siège osseux ou un siège articulaire infecté à l'intérieur du corps humain, ledit corps de confinement (1, 100, 200) comprenant une partie de base (2, 102, 202), des parois latérales (3, 103, 203) qui s'étendent à partir de ladite partie de base (2, 102, 202), ladite partie de base (2, 102, 202) et lesdites parois latérales (3, 103, 203) délimitant à l'intérieur de celles-ci au moins une cavité (4, 104, 204), et comprenant au moins une ouverture (5, 105, 205) délimitée par lesdites parois latérales (3, 103, 203) pour accéder à ladite au moins une cavité (4, 104, 204), dans laquelle ladite partie de base (2, 102, 202) et lesdites parois latérales (3, 103, 203) sont fabriquées à partir d'un matériau biologiquement compatible, du type implantable à l'intérieur du corps humain, dans lequel ladite partie de base (2, 102, 202) et/ou au moins l'une desdites parois latérales (3, 103, 203) présentent une pluralité de pores (6, 106, 206) interconnectés entre eux et/ou au moins un par ouverture, configuré de manière à placer ladite au moins une cavité (4, 104, 204) en communication avec l'extérieur dudit corps de confinement (1, 100, 200), dans lequel ladite au moins une cavité (4, 104, 204) est configurée pour être remplie d'un matériau de remplissage qui est un fluide solidifiable, et dans laquelle ladite partie de base (2, 102, 202) et lesdites parois latérales (3, 103, 203) ont une épaisseur comprise entre 0,5 mm et 20 mm.

2. Corps de confinement (1, 100, 200) selon la revendication 1, dans lequel lesdites parois latérales (3, 103, 203) s'étendent périmétriquement à partir de ladite partie de base (2, 102, 202) et/ou dans lequel lesdites parois latérales (3, 103, 203) sont continues l'une de l'autre et/ou dans lequel ladite partie de base (2, 102, 202) et lesdites parois latérales (3, 103, 203) sont façonnées de manière à correspondre substantiellement au siège osseux ou au siège articulaire ou à une partie de celui-ci dans lequel ledit corps de confinement (1, 100, 200) peut être implanté en cours d'utilisation.

3. Corps de confinement (1, 100, 200) selon la revendication 1 ou 2, dans lequel ladite au moins une ouverture (5, 105, 205) est délimitée par un bord périphérique extérieur desdites parois latérales (3, 103, 203), pour accéder à ladite au moins une cavité (4, 104, 204) et/ou dans lequel ledit corps de confinement comprend un corps de fermeture (9, 109, 209) apte à fermer ladite au moins une cavité (4, 104, 204).

4. Corps de confinement (1, 100, 200) selon la revendication 3, comprenant des moyens de contrainte pour contraindre ledit corps de fermeture (9, 109, 209) avec ledit corps de confinement (1, 100, 200).

5. Corps de confinement (1, 100, 200) selon l'une quelconque des revendications précédentes, comprenant au moins une paroi de séparation (7, 107, 207) à l'intérieur de ladite au moins une cavité (4, 104, 204), ladite au moins une paroi de séparation (7, 107, 207) faisant saillie vers le haut à partir de ladite partie de base (2, 102, 202) et/ou d'au moins une desdites parois latérales (3, 103, 203), ladite au moins une paroi de séparation (7, 107, 207) définissant au moins deux parties (4', 4", 104', 104") à l'intérieur du volume de ladite au moins une cavité (4, 104, 204).

6. Corps de confinement (1, 100, 200) selon la revendication 5, comprenant une pluralité desdites parois de séparation (7, 107, 207) configurées de manière à définir une structure sensiblement trabéculaire à l'intérieur dudit corps de confinement (1, 100, 200).

7. Corps de confinement (1, 100, 200) selon l'une quelconque des revendications précédentes, dans lequel l'épaisseur de ladite partie de base (2, 102, 202) et desdites parois latérales (3, 103, 203) est comprise entre 0,5 mm et 4 mm.

8. Dispositif d'espacement temporaire et/ou jetable (13), implantable dans le corps humain pour le traitement d'un siège osseux ou d'un siège articulaire, comprenant une partie externe (14), ayant une forme correspondant sensiblement à la forme du siège osseux ou du siège articulaire à traiter ou partie de celui-ci à laquelle ledit dispositif d'espacement (13) doit être contraint, et une partie intérieure (15), dans laquelle ladite partie extérieure (14) comprend un corps de confinement (1, 100, 200) selon l'une quelconque des revendications 1 à 7, dans laquelle l'au moins une cavité (4, 104, 204) est remplie de ladite partie intérieure (15) qui comprend un matériau de remplissage qui est un fluide solidifié, et dans laquelle ladite partie intérieure (15) comprend du ciment osseux et au moins une substance pharmaceutique ou médicale.

9. Dispositif d'espacement (13) selon la revendication 8, dans lequel ladite partie intérieure (15) est contenue dans ledit corps de confinement (1, 100, 200).

10. Dispositif d'espacement selon la revendication 8 ou 9, dans lequel ladite partie extérieure (14) comprend en outre un corps de fermeture (9, 109, 209) pour fermer ladite au moins une cavité (4, 104, 204) dudit corps de confinement (1, 100, 200), ledit corps de fermeture (9, 109, 209) ayant une première surface (10, 110), utilisé à l'extérieur de ladite au moins une cavité (4, 104, 204), et une deuxième surface (11, 111) opposée à ladite première surface (10, 110), utilisée à l'intérieur de ladite au moins une cavité (4, 104, 204) et/ou dans laquelle ledit corps de confinement (1, 100, 200) et/ou ledit corps de fermeture (9, 109, 209) comprend au moins une substance pharmaceutique ou médicale.

11. Dispositif d'espacement selon la revendication 10, dans lequel ledit corps de fermeture (9, 109, 209) est poreux, comprenant une pluralité de pores (12, 112, 212) interconnectés entre eux, et/ou une pluralité de pores (12, 112, 212) et/ou au moins une ouverture traversante, configurée de manière à placer ladite première surface (10, 110) en communication avec ladite deuxième surface (11, 111).

12. Dispositif d'espacement selon la revendication 8, dans lequel ladite partie intérieure (15) comprend au moins un noyau de renforcement (16).

13. Dispositif d'espacement selon l'une quelconque des revendications 8 à 12, comprenant au moins un biocapteur positionné à l'intérieur dudit corps de confinement (1, 100, 200), pour la détection d'au moins une grandeur physico-chimique.

14. Procédé de fabrication d'un dispositif d'espacement temporaire et/ou jetable implantable dans le corps humain pour le traitement d'un siège osseux ou d'un siège articulaire infecté selon l'une quelconque des revendications 8 à 13, comprenant les étapes de:
fournir au moins un corps de confinement (1, 100, 200) en forme de siège osseux ou de siège articulaire à traiter ou une partie de celui-ci et pourvu d'au moins une cavité (4, 104, 204), de manière à constituer une partie extérieure (14) dudit dispositif d'espacement,
fournir un matériau de remplissage,
insérer ou imprégner le matériau de remplissage dans ladite au moins une cavité (4, 104, 204),
solidifier ou façonner ledit matériau de remplissage, de manière à constituer une partie intérieure (15) dudit dispositif d'espacement.

15. Procédé selon la revendication 14, comprenant les étapes de :
fournir un corps de fermeture (9, 109, 209),
associer ledit corps de fermeture (9, 109, 209) audit corps de confinement (1, 100, 200) de manière à définir une enveloppe à l'intérieur de laquelle se trouve ladite partie intérieure (15), et/ou
contraindre ledit corps de fermeture (9, 109, 209) audit corps de confinement (1, 100, 200) par des moyens de contrainte.

16. Procédé selon la revendication 14, dans lequel ladite étape de constitution de ladite partie intérieure (15) prévoit une étape d'élimination d'un excès dudit matériau de remplissage qui dépasse dudit corps de confinement (1, 100, 200) ou comprenant une étape d'insertion d'un noyau de renfort (16) à l'intérieur de ladite partie intérieure (15) .
